# EUROPÄISCHE PATENTSCHRIFT

(11) **EP 0 722 926 B1**
(45) Veröffentlichungstag und Bekanntmachung des Hinweises auf die Patenterteilung: **27.05.1998**
(21) Anmeldenummer: 96100461.1
(22) Anmeldetag: 13.01.1996
(51) Int. Cl.: C07C 57/04, C07C 51/44

(54) **Verfahren der rektifikativen Abtrennung von (Meth)acrylsäure aus einem (Meth)acrylsäure und niedere Aldehyde enthaltenden Gemisch**
Process for rectificative separation of (meth)acrylic acid from the (meth)acrylic acid and lower aldehydes containing mixture
Procédé de séparation rectificative d'acide (méth)acrylique à partir d'un mélange renferment d'acide (méth)acrylique et des aldéhydes inférieures

(30) Priorität: 18.01.1995 DE 19501326
(43) Veröffentlichungstag der Anmeldung: 24.07.1996
(73) Patentinhaber: BASF AKTIENGESELLSCHAFT, 67056 Ludwigshafen (DE)
(72) Erfinder: Herbst, Holger, Dr., D-67227 Frankenthal (DE); Hammon, Ulrich, Dr., D-68165 Mannheim (DE)

(56) Entgegenhaltungen:
- US-A- 2 147 306

## Beschreibung

Die vorliegende Erfindung betrifft ein Verfahren der rektifikativen Abtrennung von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne.

(Meth)acrylsäure wird als verkürzte Schreibweise verwendet und steht für Acrylsäure oder Methacrylsäure. (Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich.

Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 4 405 059, EP-A 253 409, EP-A 92 097, DE-A 44 31 957 und DE-A 44 31 949).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine (Meth)acrylsäure, sondern ein Reaktionsgasgemisch erhalten, das im wesentlichen (Meth)acrylsäure, die inerten Verdünnungsgase und Nebenprodukte enthält, aus welchen die (Meth)acrylsäure abgetrennt werden muß.

Neben von (Meth)acrylsäure vergleichsweise einfach zu entfernenden und bei Folgeverwendungen der (Meth)acrylsäure weniger störenden Nebenprodukten wie z.B. Essigsäure, enthält das Reaktionsgasgemisch insbesondere auch mit (Meth)acrylsäure verwandte und daher von (Meth)acrylsäure schwerer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzlich gegebenenfalls Maleinsäureanhydrid (bezogen auf die im Reaktionsgasgemisch enthaltene Menge an (Meth)acrylsäure beträgt die Gesamtmenge dieser, bei Folgeverwendungen häufig erheblich störenden, Nebenkomponenten in der Regel ≤ 2 Gew.-%).

Üblicherweise erfolgt die Abtrennung der (Meth)acrylsäure aus dem Reaktionsgemisch über extraktive und rektifikative Trennverfahren. D.h. in der Regel wird die gebildete (Meth)acrylsäure aus dem Reaktionsgasgemisch der Gasphasenoxidation zunächst in ein geeignetes Absorptionsmittel aufgenommen. Durch rektifikative Auftrennung des Absorbats wird dann üblicherweise eine Roh-(Meth)acrylsäure erhalten, aus der mittels Durchlaufen weiterer rektifikativer Trennstufen häufig eine Rein-(Meth)acrylsäure erzeugt wird. Die Roh-(Meth)acrylsäure weist in der Regel eine Reinheit > 98 Gew.-% auf, wobei sich die Verunreinigungen insbesondere aus niederen Aldehyden und gegebenenfalls Maleinsäureanhydrid rekrutieren, wohingegen die Trennung der (Meth)acrylsäure von der Absorptionsflüssigkeit im wesentlichen quantitativ erfolgt ist. Im Unterschied zur Roh-(Meth)acrylsäure liegt die Reinheit der Rein-(Meth)acrylsäure im Normalfall bei > 99 Gew.-%.

Die DE-A 44 36 243 betrifft beispielsweise ein Verfahren zur Abtrennung von (Meth)acrylsäure aus dem Reaktionsgasgemisch der katalytischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt, dem in der Absorptionskolonne in natürlicher Weise erfolgenden Absorptionsprozeß einen Rektifikationsprozeß überlagert, indem man der Absorptionskolonne eine über ihre, aufgrund ihres Kontaktes mit der Umgebungstemperatur erfolgende, natürliche Energieabgabe hinausgehende Energiemenge entzieht, und aus dem (Meth)acrylsäure und das Absorptionsmittel (Absorbens) als Hauptbestandteile sowie niedere Aldehyde und gegebenenfalls Maleinsäureanhydrid als Nebenbestandteile enthaltenden Flüssigkeitsablauf der Absorptionskolonne (Absorbat) Roh-(Meth)acrylsäure über Kopf rektifikativ abtrennt.

Als hochsiedende inerte hydrophobe organische Flüssigkeit (Absorbens) faßt die DE-A 44 36 243 dabei alle diejenigen Flüssigkeiten zusammen, deren Siedetemperatur bei Normaldruck (1 atm) oberhalb der Siedetemperatur der (Meth)acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden.

Aus der DE-PS 21 36 396 und der DE-A 43 08 087 ist ebenfalls bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen. Das Verfahren wird im wesentlichen so durchgeführt, daß man das Reaktionsgasgemisch in einer konventionellen Absorptionskolonne zu der absteigenden Absorptionsflüssigkeit im Gegenstrom führt, danach in einer Desorptionskolonne aus den im wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponenten weitgehend entfernt und anschließend den Acrylsäure und das Absorbens als Hauptbestandteile sowie niedere Aldehyde und gegebenenfalls Maleinsäureanhydrid als Nebenbestandteile enthaltenden Flüssigkeitsablauf der Desorptionskolonne zur Abtrennung von Roh-Acrylsäure über Kopf in einer Rektifikationskolonne rektifikativ behandelt.

Die EP-A 297 445 betrifft beispielsweise ein Verfahren zur Abtrennung von Methacrylsäure aus dem Reaktionsgasgemisch der katalytischen Gasphasenoxidation durch Absorbieren der Methacrylsäure in einer mit Wasser betriebenen Absorptionskolonne. Zur Sumpfabtrennung von Roh-Methacrylsäure wird der Flüssigkeitsablauf der Absorptionskolonne rektifikativ behandelt.

Die EP-A 117 146 betrifft ein Verfahren zur Abtrennung von Acrylsäure aus dem Reaktionsgasgemisch der katalytischen Gasphasenoxidation durch Absorbieren der Acrylsäure in einer mit Wasser betriebenen Absorptionskolonne. Durch Extraktion mit Ethylacetat wird die Acrylsäure aus dem Flüssigkeitsablauf abgetrennt und aus dem Extrakt Roh-Acrylsäure über Sumpf rektifikativ gewonnen.

Die EP-B 102 642, die GB-PS 1 346 737 und die DE-B 2 207 184 betreffen die rektifikative Abtrennung von Rein-(Meth)acrylsäure aus Roh-(Meth)acrylsäure. Zur Erhöhung der Trennschärfe bezüglich niederer aldehydischer Verunreinigungen wird dabei der Zusatz von chemischen Verbindungen wie primären Aminen empfohlen, die die aldehydischen Verunreinigungen binden.

Die vorgenannten rektifikativen Abtrennungen von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne bilden lediglich einen kleinen Ausschnitt aus der im Stand der Technik aufgeführten Mannigfaltigkeit solcher Trennprobleme.

Nachteilig an all diesen rektifikativen Trennverfahren des Standes der Technik ist, unabhängig davon, ob die (Meth)acrylsäureabtrennung über Kopf oder über Sumpf erfolgt, daß sich während der rektifikativen Abtrennung, auch bei Mitverwendung von Polymerisationsinhibitoren wie Luft, Hydrochinon, Hydrochinonmonomethylether, Paranitrosophenol, Paramethoxyphenol und/oder Phenothiazin, die Rektifikationsvorrichtungen (insbesondere die Verdampferoberfläche) relativ rasch mit Belag bedecken, weshalb die üblicherweise kontinuierlich durchgeführte Rektifikation von Zeit zu Zeit zum Zwecke der Entfernung der Belagsbildung unterbrochen werden muß. Die unterschiedlichen Färbungen der Belagsbildung (schwarz und weiß), weisen aus, daß an der Belagsbildung wenigstens zwei Prozesse beteiligt sind.

Die Aufgabe der vorliegenden Erfindung bestand daher darin, ein neues Verfahren der rektifikativen Abtrennung von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne zur Verfügung zu stellen, bei dem die Belagsbildung auf den Rektifikationsvorrichtungen (insbesondere auf der Verdampferoberfläche) reduziert ist.

Demgemäß wurde ein Verfahren der rektifikativen Abtrennung von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne gefunden, das dadurch gekennzeichnet ist, daß man das die rektifikativ abzutrennende (Meth)acrylsäure umfassende Einsatzgemisch der Rektifikationskolonne nicht unmittelbar zuführt, sondern zunächst in ein brüdenseitig mit dem Verstärkerteil der Rektifikationskolonne verbundenes beheiztes Verweilzeitgefäß leitet, in dem das Einsatzgemisch am Sieden gehalten wird, und anstelle des Einsatzgemisches als solchem dem Abtriebsteil der Rektifikationskolonne die Sumpfflüssigkeit des Verweilzeitgefäßes zuführt (der unterhalb der Eintrittstelle der Sumpfflüssigkeit des Verweilzeitgefäßes gelegene Teil der Rektifikationskolonne bildet in üblicher Weise deren Abtriebsteil und der oberhalb dieser Eintrittstelle gelegene Teil der Rektifikationskolonne bildet in üblicher Weise deren Verstärkerteil).

D.h. dem Verweilzeitgefäß kommt im Rahmen des erfindungsgemäßen Verfahrens die Funktion eines Verdampfers zu, wobei der die leichter flüchtigen Komponenten angereichert aufweisende Dampf dem Verstärkerteil der Rektifikationskolonne und die die leichter flüchtigen Komponenten entreichert aufweisende Sumpfflüssigkeit dem Abtriebsteil der Rektifikationskolonne zugeführt wird. Entsprechend kann der Verweilzeitbehälter beispielsweise als Verdampfer mit Selbstumlauf, als Verdampfer mit Zwangsumlauf, als Entspannungsverdampfer, als Dünnschichtverdampfer (Sambay- oder Luva-Verdampfer) oder als Fallfilmverdampfer ausgelegt sein. Selbstverständlich kann der "Verweilzeit-Verdampfer" ein- oder mehrstufig ausgeführt sein.

Die Verweilzeit des rektifikativ aufzutrennenden Einsatzgemisches im Verweilzeitgefäß beträgt zweckmäßigerweise 0,1 bis 3 h. Um die Temperaturen in der Rektifikationskolonne möglichst gering zu halten, erfolgt die erfindungsgemäße rektifikative Abtrennung der (Meth)acrylsäure ganz allgemein vorzugsweise bei reduziertem Druck. Erfolgt die rektifikative Abtrennung der (Meth)acrylsäure dabei über Kopf- oder Seitenentnahme, wird erfindungsgemäß zweckmäßigerweise bei einem Kopfdruck ≤ 500 mbar, üblicherweise bei 10 bis 500 mbar, häufig bei 10 bis 200 mbar und vorzugsweise bei 10 bis 100 mbar gearbeitet; in entsprechender Weise betragen die zugehörigen Temperaturen im Sumpf der Rektifikationskolonne in der Regel 100 bis 230°C.

Als Rektifikationskolonne kommen für das erfindungsgemäße Verfahren alle gängigen Typen in Betracht. D.h. die Rektifikationskolonne kann z.B. eine Boden-, Füllkörper- oder Packungskolonne sein. Vorzugsweise werden Bodenkolonnen angewendet. Beispielhaft zu nennen sind Ventil-, Glocken-, Tunnel-, Sieb- und Dualflow-Bodenkolonnen. Vorzugsweise werden Glockenböden angewendet. Die Trennlinie zwischen Verstärkerteil und Abtriebsteil der Rektifikationskolonne verläuft zweckmäßig etwa am Ende des ersten Drittels der Strecke zwischen unterster und höchstgelegener theoretischer Trennstufe.

Die vorstehend beschriebene erfindungsgemäße Verfahrensweise ist das Ergebnis ausgedehnter Forschungsarbeit, im Rahmen derer nachfolgende Zusammenhänge gefunden wurden.

Zunächst wurde festgestellt, daß insbesondere an der Belagsbildung im Abtriebsteil der Rektifikationskolonne (insbesondere auf der Verdampferoberfläche) aufgrund der dort vorherrschenden erhöhten Temperaturen eine auf die aldehydischen Verunreinigungen zurückzuführende Harzbildung beteiligt ist. Durch die erfindungsgemäße Zufuhr des rektifikativ aufzutrennenden Einsatzgemisches zur Rektifikationskolonne gelangt der relativ leichtflüchtige Teil der aldehydischen Verunreinigungen über die brüdenseitige Verbindung des Verweilzeitbehälters mit dem Verstärkerteil der Rektifikationskolonne unmittelbar in selbigen Verstärkerteil, was eine Beteiligung dieser aldehydischen Verunreinigungen an der Harzbildung im Abtriebsteil unterbindet und selbige somit reduziert. Ein anderer Teil der aldehydischen Verunreinigungen verharzt bereits im Verweilzeitverdampfer. Ferner wurde gefunden, daß trotz der Stabilisierung des rektifikativ aufzutrennenden Einsatzgemisches mittels üblicher Mengen an sich üblicher Polymerisationsinhibitoren in selbigem vorab seiner Zufuhr in die Rektifikationskolonne in einem gewissen Ausmaß radikalische Oligo- und/oder Polymerisation der (Meth)acrylsäure erfolgt. Die dabei entstehenden (Meth)acrylsäureoligo- und/oder -polymerisate weisen jedoch in der Regel eine ausreichende molekulare und/oder kolloidale Löslichkeit im rektifikativ aufzutrennenden Einsatzgemisch auf, weshalb sie vorab der Zufuhr desselben in die Rektifikationskolonne normalerweise nicht sichtbar in Erscheinung treten. Aufgrund ihres erhöhten Siedepunktes reichern sich diese (Meth)acrylsäureoligc- und/oder -polymerisate innerhalb der Rektifikationskolonne jedoch zum Verdampfer hin an, um sich bei Überschreiten ihrer Löslichkeit als Belag auf den Rektifikationsvorrichtungen niederzuschlagen und auf selbigen adsorptiv fest zuhaften. Letzteres gilt insbesondere für die Verdampferoberfläche, auf welcher infolge des Verdampfens die Löslichkeitsgrenze besonders leicht überschritten wird. Durch die erfindungsgemäße Anwendung einer der Rektifikationskolonne vorgeschalteten Verweilzeitverdampferstufe wird ein Teil der im rektifikativ aufzutrennenden Einsatzgemisch in solubilisierter Form enthaltenen (Meth)acrylsäureoligo-und/oder -polymerisate aus verschiedenen Gründen bereits in der Verweilzeitverdampferstufe abgeschieden, was die Betriebsdauer der eicentlichen rektifikativen Vorrichtung verlängert (von Bedeutung ist dabei, daß die Siedetemperatur in der vorgeschalteten Verweilzeitverdampferstufe unterhalb der Temperatur im Sumpf der Rektifikationskolonne liegt).

Letzteres gilt insbesondere dann, wenn das rektifikative Verfahren die Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch betrifft, wie es z.B. im Rahmen der Abtrennung von (Meth)acrylsäure aus dem Reaktionsgasgemisch der katalytischen Gasphasenoxidation gemäß den in der DE-A 4 436 243, der DE-PS 2 136 396 und der DE-A 4 308 087 beschriebenen Verfahrensweisen auftritt. D.h. wenn das (Meth)acrylsäure und eine höher als (Meth)acrylsäure siedende inerte hydrophobe organische Flüssigkeit als Hauptbestandteile sowie niedere Aldehyde als Nebenbestandteile enthaltende Einsatzgemisch für das erfindungsgemäße Verfahren z.B. aus den Reaktionsgasgemischen der katalytischen Gasphasenoxidation als Flüssigkeitsablauf einer Gegenstromabsorption mit anschließender Desorption durch Abstreifen gemäß der DE-PS 2 136 396 oder der DE-A 4 308 087 oder als Flüssigkeitsablauf einer Gegenstromabsorption mit überlagerter Rektifikation gemäß der DE-A 4 436 243 erhalten wurde. Unter hochsiedender inerter hydrophober organischer Flüssigkeit sind hier solche Flüssigkeiten zu verstehen, deren Siedepunkt bei Normaldruck (1 atm) oberhalb der Siedetemperatur der (Meth)acrylsäure liegt und in denen die Löslichkeit (Gew.-%, bezogen auf das Gewicht der Lösung) von (Meth)acrylsäureoligo- und/oder -polymerisaten bei 25°C und 1 atm geringer als in reiner (Meth)acrylsäure ist.

Insbesondere sind es solche hochsiedenden organischen Flüssigkeiten, die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Im engeren Sinn umfaßt der Begriff hier die hochsiedenden organischen Absorptionsflüssigkeiten, die in der DE-PS 2 136 396, der DE-A 4 308 087 sowie der DE-A 4 362 243 empfohlen werden. Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl, oder Mischungen der vorgenannten Flüssigkeiten wie z.B. ein Gemisch aus 70 bis 75 Gew.-% Diphenylether und 25 bis 30 Gew.-% Diphenyl. Eine günstige hochsiedende hydrophobe organische Absorptionsflüssigkeit ist auch ein Gemisch, bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat.

Im Fall der Methacrylsäure kann dabei die gasphasenkatalytisch oxidative Herstellung z.B. ausgehend von Methacrolein erfolgt sein, das seinerseits durch gasphasenkatalytische Oxidation von tert.-Butanol, iso-Butan oder iso-Buten oder durch Umsetzung von Formaldehyd mit Propionaldehyd gemäß der EP-B 92 097 oder der EP-B 58 927 erhalten worden sein kann. Häufig erfolgt die gasphasenkatalytische Oxidation des tert.-Butanols, iso-Butans oder iso-Butens unter Verwendung einer katalytisch aktiven Masse der allgemeinen Formel I

Mo₁₂BiₐFe_{b}X¹_{c}X²_{d}X³ₑX⁴_{f}Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 30,
- d: 0,02 bis 2,
- e: 0 bis 25,
- f: 0 bis 10 und
- n: eine ganze Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der DE-A 4 023 239. Das dabei anfallende Methacrolein wird in der Regel ohne Zwischenreinigung zur Weiteroxidation verwendet. Dabei kann die gasphasenkatalytische Oxidation des Methacroleins, vom speziellen Temperaturverlauf abgesehen, gemäß den Ausführungen der DE-A 4 132 263 bei Temperaturen von 200 bis 350°C bzw. gemäß der DE-A 4 132 684 bei Temperaturen von 250 bis 400°C erfolgen. Insbesondere können dabei die in der DE-A 4 022 212 aufgeführten Multimetalloxidkatalysatoren angewendet werden.

Im Fall von Acrylsäure kann die gasphasenkatalytisch oxidative Herstellung z.B. einstufig ausgehend von Acrolein oder zweistufig ausgehend von Propylen über Acrolein erfolgt sein.

Als Multimetalloxidkatalysatoren kommen dabei für die katalytische Gasphasenoxidation des Propylens, insbesondere solche der allgemeinen Formel II

Mo₁₂BiₐFe_{b}X¹_{c}X²_{d}X³ₑX⁴_{f}Oₙ (II),

in der die Variablen nachfolgende Bedeutung aufweisen:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente bestimmt wird, und
für die katalytische Gasphasenoxidation des Acroleins, insbesondere solche der allgemeinen Formel III

Mo₁₂VₐW_{b}Cu_{c}Ni_{d}X¹ₑX²_{f}X³_{g}X⁴ₕX⁵ᵢOₙ (III),

in der die Variablen nachfolgende Bedeutung aufweisen:
- X¹: ein oder mehrere Alkalimetalle,
- X²: ein oder mehrere Erdalkalimetalle,
- X³: Chrom, Mangan, Cer und/oder Niob,
- X⁴: Antimon und/oder Wismut,
- X⁵: Silicium, Aluminium, Titan und/oder Zirkonium,
- a: 1 bis 6,
- b: 0,2 bis 4,
- c: 0,5 bis 6,
- d: 0,2 bis 6,
- e: 0 bis 2,
- f: 0 bis 3,
- g: 0 bis 5,
- h: 0 bis 40,
- i: 0 bis 40 und
- n: eine ganze Zahl, die durch die Wertigkeit und Häufigkeit der von Sauerstoff verschiedenen Elemente bestimmt wird,
in Betracht. Die Reaktionsgase der ersten Oxidationsstufe werden üblicherweise ohne Zwischenreinigung der zweiten Oxidationsstufe zugeführt. Die üblicherweise angewendeten Reaktionsbedingungen können z.B. der DE-A 4 431 957 sowie der DE-A 4 431 949 entnommen werden.

In der Regel enthält ein solches, vorstehend beschriebenes, im wesentlichen aus (Meth)acrylsäure und einer höher als (Meth)acrylsäure siedenden inerten hydrophoben organischen Flüssigkeit als Hauptbestandteilen sowie niederen Aldehyden als Nebenbestandteilen bestehendes, Gemisch 5 bis 25, meist 5 bis 15 Gew.-% (Meth)acrylsäure.

Selbstverständlich wird das erfindungsgemäße Verfahren im Beisein an sich üblicher Mengen von an sich üblichen Polymerisationsinhibitoren durchgeführt. Vorzugsweise wird Phenothiazin als Polymerisationsinhibitor angewendet. Normalerweise werden die Polymerisationsinhibitoren auf die (Meth)acrylsäuremenge (Gewicht) bezogen in Mengen von 50 bis 1000 ppm eingesetzt. Ferner ist es aufgrund der die Polymerisation von (Meth)acrylsäure inhibierenden Wirkung von Luftsauerstoff vorteilhaft, die Rektifikationskolonne luftdurchströmt zu betreiben.

Selbstverständlich kann das erfindungsgemäße Verfahren auch mit anderen Verfahren des Standes der Technik zur Unterdrückung von Belagsbildung gekoppelt angewendet werden.

Im Rahmen des erfindungsgemäßen Verfahrens muß selbstredend der Verweilzeitverdampfer von Zeit zu Zeit einer Belagsentfernung unterzogen werden. Durch Umschaltung auf einen zweiten Verweilzeitverdampfer kann dabei der Betrieb der Rektifikationskolonne jedoch aufrechterhalten werden.

### Beispiele

(Es wurde in Anwesenheit von 200 ppm (bezogen auf das Gewicht der Acrylsäure) Phenothiazin als Polymerisationsinhibitor gearbeitet).
a) Durch katalytische Gasphasenoxidation von Acrolein gemäß Beispiel B1 der DE-A 4 302 991 wurde ein Acrylsäure enthaltendes Reaktionsgasgemisch erzeugt. 2,1 Nm³/l dieses Reaktionsgasgemisches wurden in einem Gaskühler durch Einspritzen eines Kühlmittelgemisches aus 57,4 Gew.-% Diphenylether, 20,7 Gew.-% Diphenyl und 20 Gew.-% o-Dimethylphthalat auf 170°C gekühlt.
   Anschließend wurde in einem Abscheider der flüssig gebliebene Anteil des Kühlmittels von der aus Reaktionsgas und verdampftem Kühlmittel bestehenden Gasphase abgetrennt. Die eine Temperatur von 170°C aufweisende Gasphase wurde unterhalb des ersten Bodens in eine Glockenbodenkolonne mit 27 Böden eines Durchmessers von 80 mm geleitet und dem Gegenstrom von 3 l/h des gleichfalls aus 57,4 Gew.-% Diphenylether, 20,7 Gew.-% Diphenyl und 20 Gew.-% o-Dimethylphthalat zusammengesetzten, am Kolonnenkopf mit einer Temperatur von 45°C aufgegebenen, Absorptionsmittels ausgesetzt. Der Ablauf der Absorptionskolonne wurde in einem Wärmetauscher indirekt auf 105°C erwärmt und auf den Kopf einer Desorptionskolonne gegeben, die als Glockenbodenkolonne mit 20 Böden ausgeführt war. In der Desorptionskolonne wurden im Vergleich zu Acrylsäure leichtsiedende Komponenten wie Essigsäure durch Strippen mit Stickstoff (400 l/h, Gegenstrom) weitgehend aus dem ansonsten Acrylsäure/niedere Aldehyde/Absorptionsmittel enthaltenden Gemisch entfernt. Der Ablauf der Desorptionskolonne bestand aus
   84,5 Gew.-% Absorptionsmittel,
   15 Gew.-% Acrylsäure und
   insbesondere niederen Aldehyden als Nebenkomponenten.
   Er wurde mit einer Temperatur von 25°C in einer Menge von 3 l/h zwischen dem fünften und sechsten Boden (vom Verdampfer aus betrachtet) in eine 20 Glockenböden umfassende, luftdurchströmte, Rektifikationskolonne (Durchmesser der Kolonne: 80 mm) geleitet. Die Rektifikationskolonne wurde mit einer Sumpftemperatur von 160°C und einem Sumpfdruck von 130 mbar sowie einem Kopfdruck von 80 mbar betrieben.
   Zwischen dem fünfzehnten und sechszehnten Boden (vom Verdampfer aus betrachtet) wurden über Seitenabzug pro Stunde 60 ml Acrylsäure in einer Reinheit von 99,3 Gew.-% kontinuierlich entnommen. Das dampfförmige Kopfprodukt wurde kondensiert, mit Polymerisationsinhibitor versetzt und bis auf eine Entnahmemenge von 5 ml/h oberhalb des obersten Glockenbodens wieder in die Rektifikationskolonne zurückgeführt.
   Nach einer Betriebsdauer von 165 h mußte der Betrieb der Rektifikationskolonne infolge zu starker Belagsbildung im Abtriebsteil der Rektifikationskolonne unterbrochen werden.
b) Wie a), der Ablauf der Desorptionskolonne wurde jedoch zunächst in einen Volumeninhalt von 500 ml aufweisenden beheizbaren Rundkolben geführt, in selbigem auf eine Temperatur von 150°C erwärmt und bei einer Verweilzeit von 0,5 h und einem Arbeitsvolumen von 250 ml über einen unteren Ablauf des Rundkolbens zwischen dem fünften und sechsten Boden (vom Verdampfer aus betrachtet) der Rektifikationskolonne zugeführt. Nach einer Betriebsdauer von 167 h mußte der Betrieb der Rektifikationskolonne abgebrochen werden. Auf der Innenfläche der Verweilzeit-Rundkolbens war im wesentlichen keine Belagsbildung festzustellen.
c) Wie b), der Verweilzeit-Rundkolben wurde jedoch über ein Brüdenrohr unmittelbar oberhalb des untersten Glockenbodens des Verstärkerteils mit dem Verstärkerteil der Rektifikationskolonne verbunden. Die Betriebsdauer der Rektifikationskolonne bis zum erforderlichen Abbruch verlängerte sich auf 276 h. Auf der Innenfläche des Verweilzeit-Rundkolbens trat Belagsbildung auf.

## Patentansprüche

1. Verfahren der rektifikativen Abtrennung von (Meth)acrylsäure aus einem (Meth)acrylsäure als Hauptbestandteil und niedere Aldehyde als Nebenbestandteile enthaltenden Gemisch in einer aus Abtriebsteil und Verstärkerteil bestehenden Rektifikationskolonne, dadurch gekennzeichnet, daß man das die rektifikativ abzutrennende (Meth)acrylsäure umfassende Einsatzgemisch der Rektifikationskolonne nicht unmittelbar zuführt, sondern zunächst in ein brüdenseitig mit dem Verstärkerteil der Rektifikationskolonne verbundenes beheiztes Verweilzeitgefäß leitet, in dem das Einsatzgemisch am Sieden gehalten wird und anstelle des Einsatzgemisches als solchem dem Abtriebsteil der Rektifikationskolonne die Sumpfflüssigkeit des Verweilzeitgefäßes zuführt.

## Claims

1. A process for the isolation of (meth)acrylic acid from a mixture containing (meth)acrylic acid as the main component and lower aldehydes as secondary components by rectification in a rectification column consisting of a stripping section and a rectification section, wherein the starting mixture comprising the (meth)acrylic acid to be isolated by rectification is not fed directly to the rectification column but is first passed into a heated dwell vessel which is connected to the vapor side of the rectification section of the rectification column and in which the starting mixture is kept at the boil and, instead of the starting mixture as such, the bottom liquid of the dwell vessel is fed to the stripping section of the rectification column.

## Revendications

1. Procédé de séparation par rectification d'acide (méth)acrylique à partir d'un mélange contenant de l'acide (méth)acrylique en tant que constituant principal et des aldéhydes inférieurs en tant que constituants secondaires, dans une colonne de rectification composée d'une section d'entraînement et d'une section de concentration, caractérisé en ce que l'on introduit le mélange de charge contenant l'acide (méth)acrylique à séparer par rectification non pas directement dans la colonne de rectification, mais d'abord dans un récipient de séjour chauffé raccordé par le côté à la section de concentration de la colonne de rectification, dans lequel le mélange de charge est maintenu à ébullition et l'on introduit dans la section d'entraînement de la colonne de rectification le liquide de fond du récipient de séjour, au lieu du mélange de charge en tant que tel.
